# EUROPEAN PATENT APPLICATION

(11) **EP 3 432 309 A1**
(43) Date of publication of application: **23.01.2019**
(21) Application number: 17182651.4
(22) Date of filing: 21.07.2017
(51) Int. Cl.: G16H 10/20

(54) **PROVIDING A FAILURE PARAMETER IN MEDICAL CLOUD INFRASTRUC-TURES**

(71) Applicant: Siemens Healthcare GmbH, 91052 Erlangen (DE)
(72) Inventor: Ertel, Emilian, 90480 Nürnberg (DE); Ukis, Vladyslav, 90489 Nürnberg (DE)

(57) **Abstract**

In one aspect the invention relates to a method for providing a failure parameter, comprising the step of receiving a medical data record by a first application module; furthermore comprising the step of determining a failure record by the first application module, if the medical data record cannot be processed by the first application module, wherein the failure record is based on the data record; furthermore comprising the step of transmitting the failure record from the first application module to a failure analysis module; furthermore comprising the step of saving the failure record in a failure database by the failure analysis module; furthermore comprising the step of determining a failure parameter by the failure analysis module, wherein the failure parameter is based on a statistical analysis of the failure database; furthermore comprising the step of providing the failure parameter by the failure analysis module.

## Description

In common cloud infrastructures data records are transmitted between various modules as well processed by these modules in complicated workflows. In each of these transmitting and processing steps errors can occur, which make the data records non processible for some of the modules of the cloud infrastructure. Examples for such errors are invalid or corrupt file formats, or the business logic of some application programs rejecting data records as invalid. As a result, some calculation results (in particular results of a statistical analysis) may differ from the expectation of the user, because data records that cannot be processed at any stage of the workflow in the cloud cannot be taken into account in the calculation.

This problem is particularly relevant within processing medical data. In the medical cloud platform teamplay it is common to store anonymized examination results in the cloud and to perform a statistical analysis using this stored results, e.g. the average time of the medical examination, the average radiation dose absorbed by the patient due to the examination, or the number of radiation does outliers.

The data which is stored in the cloud originates from information systems located in the hospital, e.g. a PACS (acronym for "Picture Archiving and Communication System"), a HIS (acronym for "Hospital Information System"), a LIS (acronym for "Laboratory Information System") or a RIS (acronym for "Radiology Information System"). If there are errors in the data processing of the cloud workflow, the basic set for the statistical analysis differs from the set of medical data records within the local systems. This leads to incorrect data analysis results. Furthermore, it is unclear for an inexperienced user of the system why there is a difference between the basic sets at all.

So is the object of the present invention to take the erroneous data records into account for the medical data analysis in order to improve the medical data analysis.

This object is solved by a method according to claim 1, a providing system according to claim 12, a computer program product according to claim 14 and a computer-readable storage medium according to claim 15.

In the following the solution according to the invention is described with respect to the claimed providing system as well as with respect to the claimed method. Features, advantages or alternative embodiments herein can be assigned to the other claimed objects and vice versa. In other words, claims for the providing systems can be improved with features described or claimed in the context of the method. In this case, the functional features of the method are embodied by objective units of the providing system.

In one aspect the invention relates to a method for providing a failure parameter, comprising the step of receiving a medical data record by a first application module; furthermore comprising the step of determining a failure record by the first application module, if the medical data record cannot be processed by the first application module, wherein the failure record is based on the data record; furthermore comprising the step of transmitting the failure record from the first application module to a failure analysis module; furthermore comprising the step of saving the failure record in a failure database by the failure analysis module; furthermore comprising the step of determining a failure parameter by the failure analysis module, wherein the failure parameter is based on a statistical analysis of the failure database; furthermore comprising the step of providing the failure parameter by the failure analysis module. In particular, the failure parameter is based on a set of failure parameters stored within the failure database. In particular, the failure record can be identical with the medical data record.

The inventor has recognized that by storing failure records within a failure database information about the number of errors and about reasons for the errors can be provided by the failure analysis module in terms of a failure parameter. This failure parameter can then be used for refining the analysis of the medical data.

In particular, the step of transmitting the failure record from the first application module to the second application module comprises the steps of sending the failure record by the first application module and the step of receiving the failure record by the failure analysis module. In particular, the step of receiving the failure record is executed by an input interface of the failure analysis module. In particular, the step of saving the failure record is executed by a storage unit of the failure analysis module. In particular, the step of determining the failure parameter is executed by a calculation module of the failure analysis module. In particular, the step of providing the failure parameter is executed by an output interface of the failure analysis module.

In another possible aspect of the invention, the failure record can be altered by the failure analysis module prior to storing in the failure database. The inventors have recognized that using a preprocessing of the failure record its data structure can be adapted to storage or computing time restrictions.

In another aspect of the invention relates to a method for providing a data analysis, comprising all steps of the method for providing a failure parameter, furthermore comprising the steps of receiving the failure parameter by a second application module; and furthermore comprising the step of providing a medical data analysis based on the failure parameter by the second application module. The first application module and be different from the second application module, but the first application module can also be identical with the second application module.

According to a further aspect of the invention the failure record comprises a failure type, wherein the failure type indicates the reason for the medical data record being non-processible by the first application module. The inventors have recognized that by providing the failure type the information about the reason of the failure to process a medical data record can be stored and made accessible for further analysis.

According to a further aspect of the invention the medical data record comprises the result of a medical examination of a patient by a medical apparatus, wherein the failure record is an anonymized data record. In particular, the failure record comprises metadata of the data record, and the failure record does not comprise personal data of the patient. The inventors have recognized that by using only anonymized failure records the failure analysis module does not need to handle security and/or privacy restrictions, which makes it easier to implement and to use the failure analysis module.

According to a further aspect of the invention the medical data record comprises the result of a medical examination of a patient by a medical apparatus, wherein the failure record comprises metadata, and wherein the metadata relates to the medical apparatus, to a parameter of the medical examination and/or to the first application module. In particular the metadata does not comprise personal data or examination results. In particular, a parameter of the medical examination is an input value for the medical apparatus which influences the result of the medical examination. In particular, a parameter of the medical examination is not a result of the medical examination. In particular, the medical examination is a medical imaging examination. The inventors have recognized that by including metadata relating to the medical apparatus and/or to parameters of the medical examination, medical apparatuses and/or certain parameters of medical examinations that lead to erroneous data records can be identified quickly and easily.

According to a further aspect of the invention the metadata contains at least one of the following values of the medical examination:
- start time and/or end time of the medical examination,
- duration of the medical examination,
- input parameter of the medical examination,
- hardware components of the medical apparatus,
- software version of the medical apparatus,
- identifier of the first application module.
In particular, an input parameter of the medical examination can be an imaging protocol of the medical examination, if the medical examination is a medical imaging examination. The inventors have recognized that these values can be used for identifying medical examination procedures or medical apparatuses which lead to erroneous data records.

According to a further aspect of the invention the medical data record cannot be processed by the first application module due to the medical data record exhibiting a non-readable format or due to the medical data record being classified as inconsistent by the first application module. In particular, the medical data record exhibits a non-readable data format, if the medical data record is incomplete (e.g. to errors in the creation or the transmission of the medical data record), of if the first medical application is embodies to process only data records with a different data format and/or a different version of the data format. In other words, a medical data record with a non-readable data format cannot be processed by the first application module at all. In particular, a medical data record is classified as inconsistent if at least one value of the medical data record (e.g. the duration of the medical examination or the radiation dose of the medical examination) lies outside a specified region or interval of this value. Such regions or intervals are usually specified in the first application module to prevent medical data records not relating to a medical examination to be processed. In other words, a medical data record is classified as inconsistent, if the medical data record can theoretically be processed by the first application module, but is rejected according to some program logic of the first application module. The inventors have recognized that these two reasons for the first application module not being able to process the medical data record cover a significant majority of all possible reasons, so restricting to these two reasons leads to a simpler but yet precise method for error management.

According to a further aspect of the invention the failure parameter relates to the number of failure records in the failure database. The inventors have recognized that this number can be calculated very fast and efficiently, and gives valuable insight into the total defectiveness of the system at the same time.

According to a further aspect of the invention the failure record comprises a module identifier of the first application module, wherein the failure parameter comprises the number of failure records in the failure database comprising a given module identifier. In particular, the failure parameter can comprise a list of module identifiers and the number of failure records comprising the respective module identifier. The inventors have recognized that by including the module identifier into the failure record and by resting the failure parameter on the module identifier an erroneous first application module can be identified quickly and easily.

According to a further aspect of the invention the medical data record comprises the results of a medical examination by an medical apparatus, wherein the failure record comprises an apparatus identifier of the medical apparatus, and wherein the failure parameter comprises the number of failure records in the failure database comprising a given apparatus identifier. In particular, the failure parameter can comprise a list of apparatus identifiers and the number of failure records comprising the respective apparatus identifier. The inventors have recognized that by including the apparatus identifier into the failure record and by resting the failure parameter on the module identifier an erroneous medical apparatus can be identified quickly and easily.

According to a further possible aspect of the invention the medical data record comprises the results of a medical examination by a medical apparatus, wherein the failure record comprises a module identifier and an apparatus identifier of the medical apparatus, and wherein the failure parameter comprises the number of failure records comprising a given module identifier and a given apparatus identifier. In particular, the failure parameter can comprise a list of module identifiers, apparatus identifiers and the number of failure records comprising the respective module identifier and the respective apparatus identifier.

In another aspect the invention relates to a providing system, comprising the following units:
- first application module, embodied for receiving a medical data record,
   furthermore embodied for determining a failure record, if the medical data record cannot be processed by the first application module, wherein the failure record is based on the data record,
   furthermore embodied for transmitting the failure record to a failure analysis module
- failure analysis module, embodied for saving the failure record in a failure database,
   furthermore embodied for determining a failure parameter, wherein the failure parameter is based on a statistical analysis of the failure database,
   furthermore embodied for providing the failure parameter.

According to a further aspect of the invention the providing system comprises the following unit:
- second application module, embodied for receiving (REC-2) the failure parameter,
   furthermore embodied for providing (PROV-2) a medical data analysis based on the failure parameter.

In particular the providing system can be embodied to execute the method according to the invention and its aspects. The providing system is embodied to execute the method its aspects by the first application module, the failure analysis module and the optional second application module being embodied to execute the respective method steps.

The providing system can be realized as a data processing system or as a part of a data processing system. Such a data processing system can, for example, comprise a cloud-computing system, a computer network, a computer, a tablet computer, a smartphone or the like. The providing system can comprise hardware and/or software. The hardware can be, for example, a processor system, a memory system and combinations thereof. The hardware can be configurable by the software and/or be operable by the software.

A possible further aspect of the invention relates to the providing system, wherein the failure analysis module comprises a first failure analysis sub-module and a second failure analysis sub-module, wherein the failure database comprises a first failure sub-database and a second failure sub-database, wherein the first failure analysis sub-module and the first failure sub-database are located in a hospital environment, and wherein the second failure analysis module and the second failure sub-database are located in a public cloud environment. In particular, both the first failure analysis sub-module and the first failure sub-database are embodied as the failure analysis module and the failure database according to one of the aspects of the invention. In particular, both the second failure analysis sub-module and the second failure sub-database are embodied as the failure analysis module and the failure database according to one of the aspects of the invention. The inventors have recognized that by using a separate module within the private hospital environment and within the public cloud environment different failure records can be saved in different environments, so that data privacy regulations can be fulfilled.

The invention relates in one aspect to a computer program product comprising a computer program, the computer program being loadable into a storage unit of a providing system, including program code sections to make the providing system execute the method according to an aspect of the invention when the computer program is executed in said providing system.

The invention relates in one aspect to a computer-readable medium, on which program code sections of a computer program are saved, said program code sections being loadable into and/or executable in a providing system to make the providing system execute the method according to an aspect of the invention when the program code sections are executed in providing system.

The realization of the invention by a computer program product and/or a computer-readable medium has the advantage that already existing providing systems can be easily adopted by software updates in order to work as proposed by the invention.

The computer program product can be, for example, a computer program or comprise another element apart from the computer program. This other element can be hardware, for example a memory device, on which the computer program is stored, a hardware key for using the computer program and the like, and/or software, for example a documentation or a software key for using the computer program.

The invention relates in another possible aspect to a first application module, embodied for receiving a medical data record, furthermore embodied for determining a failure record, if the medical data record cannot be processed by the first application module, wherein the failure record is based on the data record, furthermore embodied for transmitting the failure record to a failure analysis module.

The invention relates in another possible aspect to a failure analysis module, embodied for saving the failure record received from the first application module in a failure database, furthermore embodied for determining a failure parameter, wherein the failure parameter is based on a statistical analysis of the failure database, furthermore embodied for providing the failure parameter, in particular embodied for providing the failure parameter to a second application module.

The invention relates in another possible aspect to a second application module, embodied for receiving the failure parameter, in particular embodied for receiving the failure parameter from a failure analysis module, furthermore embodied for providing a medical data analysis based on the failure parameter.

A medical data record can comprise data relating to a medical examination, in particular personal data of the patient examined (e.g. the patient name, the patient age, the patient sex), medical examination results (e.g. images or laboratory values), and metadata of the examination. In particular, the medical data record can be formatted according to a medical data format, e.g. DICOM (acronym for "Digital Imaging and Communications in Medicine") or HL7 (acronym for "Health Level Seven").

Metadata of a medical examination or a respective medical data record can comprise all data which is not related to the patient of the medical examination and which is not related to the result of the medical examination. For example, the metadata of a medical examination can comprise an identifier of the medical apparatus used for the medical examination, the procedure used for the medical examination (e.g. the imaging protocol, or the workflow within the laboratory for a sample), the start time, the end time and/or the duration of the medical examination, a radiologic dose administered by the medical examination. In particular, metadata relates to the medical apparatus, a parameter of the medical examination and/or the first application module.

A failure record is usually based on a medical data record, and relates to an error occurred during the processing of the medical data record. A failure record can comprise the medical data record in whole or in part. Furthermore, a failure record can also comprise additional data or parameters; in particular data related with the error occurred during the processing of the medical data record.

The private hospital environment (or short, the hospital environment, or the hospital IT infrastructure) is particularly the IT infrastructure located directly at a hospital, which can be accessed only by local users and by means of remote maintenance. The private hospital environment can comprise medical modalities, servers (e.g. for data storage and for computations), clients and the network connecting different entities, as well as software programs running on these hardware components. The private hospital environment can be identified with an intranet. The private hospital environment can comprise components which are embodied to receive data from outside the private hospital environment or to send data to the outside of the private hospital environment; usually these components are embodied to prevent unauthorized access to the private hospital environment.

The public cloud environment (or short the cloud environment, or the cloud IT infrastructure) is particularly the IT infrastructure located in the cloud, embodied to communicate and interact with one or several private hospital environments. The public cloud infrastructure can comprise servers (e.g. for data storage and for computations), clients and the network connecting different entities, as well as software programs running on these hardware components. The private hospital environment can be a part of the internet. Usually the public cloud environment is embodied to allow access only to authorized users.

A cloud or a cloud environment comprises computing services and/or storage services made available by a service provider, which can be used by one or several service users according to their demand. In order to make these services available, the service provider usually operates physical computing units (e.g. comprising microprocessors) and storage units (e.g. comprising hard disks), which can then be used by the service providers by predefined interfaces. Such interfaces can relate on the usage of virtual machines or API calls. Known variants of a cloud environment comprise IaaS (acronym for "Infrastructure as a Service"), PaaS (acronym for "Platform as a Service") or SaaS (acronym for "Software as a Service").
- Fig. 1: shows a flow chart of an embodiment of the method for providing a failure parameter,
- Fig. 2: shows a flow chart of an embodiment of the method for providing a data analysis,
- Fig. 3: shows an embodiment of a providing system,
- Fig. 4: shows a medical cloud data infrastructure without a providing system,
- Fig. 5: shows the medical cloud data infrastructure with an embodiment of a providing system, and
- Fig. 6: shows the medical cloud data infrastructure with another embodiment of a providing system.

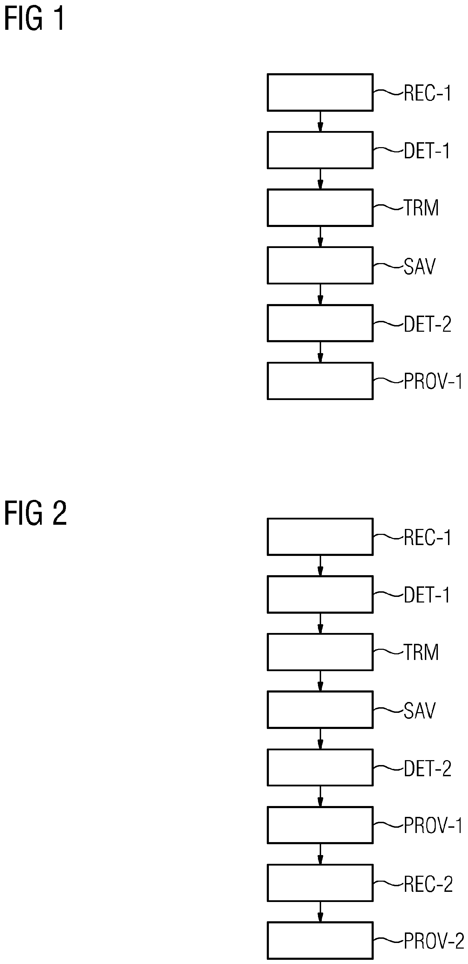

Fig. 1 shows a flow chart of an embodiment of the method for providing a failure parameter. The first step of the displayed embodiment is receiving REC-1 a medical data record by a first application module 320. In this embodiment, the first application module 320 is an instance of a software application, which provides a software interface for receiving medical data records. In this embodiment, a medical data record is the result of a medical imaging examination with a medical imaging apparatus, here a computed tomography scanner. Alternatively, the medical imaging apparatus can be a magnetic resonance scanner, an X-ray scanner or an ultrasound scanner. The medical data record can also be the result of another medical examination, e.g. laboratory results or results from medical apparatuses for point-of-care diagnostics (e.g. measuring blood glucose, temperature, oxygen saturation of the blood, heartbeat sequence, etc.). The medical data record can also be a patient data record e.g. from a hospital information system, comprising personal data and results from previous examinations.

The second step of the displayed embodiment is determining DET-1 a failure record 341.1, 341.2 by the first application module 320, if the medical data record cannot be processed by the first application module 320. In this embodiment the failure record 341.1, 341.2 comprises an identifier 342.1, 342.2 of the first application module 320, an identifier 343.1, 343.2 of the medical apparatus, and a failure type 344.1, 344.2 that indicates the reason the medical data record cannot be processed by the first application module 320.

The identifier 342.1, 342.2 of the first application module 320 comprises in this embodiment the name of the software program and the version of the software program in a string, for example "ABC-Software v1.1", where "ABC-Software" is the name of the software program and "v1.1" is the version of the software program. The identifier 343.1, 343.2 of the medical apparatus comprises a serial number of the medical apparatus. In particular, the identifier 343.1, 343.2 of the medical apparatus can also comprise the hardware components used in the medical apparatus and/or the version of the operating software of the medical apparatus.

In this embodiment the failure type 344.1, 344.2 is a string comprising an error message. In this embodiment two reasons for non-processability are treated, the first one is that the medical data record is corrupt or incomplete (error message "DATA_CORRUPT"), the second one is that the medical data record does not comply with the rules for a valid medical data record (error message "DATA_INVALID"). In this embodiment, a medical data record does not comply with the rules if certain parameters of the medical data record lie outside predefined boundaries, or if certain conditions are met or not met. E.g. some post-processing algorithms overwrite the start and the end time of the examination within a DICOM-file with times from the post-processing, in this case it is not possible to evaluate this data for obtaining usage statistics about medical apparatuses. Alternatively it is possible to use more granular error messages which describe the reason for non-processability in more detail (e.g. the error message "DATA_INVALID_EXAMINATION_TIME" for an examination time outside of the valid interval for an examination time, or "DATA_INVALID_ANONYMIYATION" for a non-anonymized medical data record).

The third step of this embodiment is transmitting TRM the failure record 341.1, 341.2 from the first application module 320 to a failure analysis module 330. In particular, the failure record 341.1, 341.2 is transmitted to an input interface 331 of the failure analysis module 330. Since the failure record 341.1, 341.2 in this embodiment is a complex data type comprising several data fields (e.g. the identifier 342.1, 342.2 of the first application module 320, for the identifier 343.1, 343.2 of the medical apparatus, and for the failure type 344.1, 344.2), the failure record 341.1, 341.2 has to be serialized before transmitting TRM it from the first application module 320 to the failure analysis module 330, or alternatively stored in a common data format (e.g. XML).

The fourth step of this embodiment is saving SAV the failure record 341.1, 341.2 in a failure database 340 by the failure analysis module 330. The failure records 341.1, 341.2 are stored in a relational database (e.g. using SQL commands), but they can also be stored in a non-relational database or as single files on a hard drive.

The fifth step of this embodiment is determining DET-2 a failure parameter by the failure analysis module 330, wherein the failure parameter is based on a statistical analysis of the failure database 340. In this embodiment a statistical analysis is based on counting the number of failure records 341.1, 341.2 with a certain property. One can also use more complicated statistical analysis methods, e.g. calculating means, variances or frequencies. In this embodiment, the statistical analysis results in a list of module identifiers 342.1, 342.2, apparatus identifiers 343.1, 343.2 and the number of failure records 341.1, 341.2 in the failure database 340 comprising the respective module identifier 342.1, 342.2 and the respective apparatus identifier 343.1, 343.2.

Suppose that there are two first application modules 320 with module identifier "module_1" and "module_2", and two medical apparatuses with apparatus identifier "apparatus_1" and "apparatus_2". The resulting list is then
- ("module_1", "apparatus_1"): "number_1_1"
- ("module_1", "apparatus_2"): "number_1_2"
- ("module_2", "apparatus_1"): "number_2_1"
- ("module_2", "apparatus_2"): "number_2_2"
where "number_i_j" is the number of failure records 341.1, 341.2 in the failure database 340 comprising the module identifier "module_i" and the apparatus identifier "apparatus_j". So if the failure database 340 has the following entries
- ("module_1", "apparatus_1", "DATA_CORRUPT")
- ("module_1", "apparatus_1", "DATA_CORRUPT")
- ("module_2", "apparatus_1", "DATA_INVALID")
- ("module_2", "apparatus_2", "DATA_INVALID")
then the resulting list of failure parameters is:
- ("module_1", "apparatus_1"): 2
- ("module_1", "apparatus_2"): 0
- ("module_2", "apparatus_1"): 1
- ("module_2", "apparatus_2"): 1

Optionally, the failure records 341.1, 341.2 can comprise other metadata of the medical examination, e.g. the duration of the medical examination, the starting time of the medical examination, the type of the examination protocol used for the medical examination, or the name of the medical professional executing the medical examination. In this case, the failure parameter can comprise this additional metadata, or also analysis with respect to this metadata. For example there might be a correlation between the examination protocol used and the invalidity of the medical data record, which can be detected using statistical analysis of the failure parameter.

The last step of this embodiment is providing PROV-1 the failure parameter by the failure analysis module 330. In particular, the failure parameter can be provided by an output interface 332 of the failure analysis module 330. In this embodiment, the providing PROV-1 comprises sending the failure parameter to a second application module 350, wherein the sending is executed in answer to an API (acronym for "Application Programming Interface") call by the second application module 350. Alternatively the failure parameter can also be stored in a storage which is externally available, e.g. on a web-server (which can be accessed e.g. by a web-service or a HTTP-request).

Fig. 2 shows a flow chart of an embodiment of the method for providing a data analysis. The steps of receiving REC-1, determining DET-1, transmitting TRM, saving SAV, determining DET-2 and providing PROV-1 are the same as in the embodiment of the method for providing a failure parameter displayed and explained in Fig. 1.

The seventh step of the embodiment of the method for providing a data analysis is receiving REC-2 the failure parameter by the second application module 350. In this embodiment, the first application module 320 and the second application module 350 are not identically; alternatively the first application module 320 and the second application module 350 can be identically. Furthermore, here the step of receiving REC-2 is executed as an API call to the failure analysis module 330 and the subsequent receiving of the answer to the API call.

The last step of the embodiment of the method for providing a data analysis is providing PROV-2 a medical data analysis based on the failure parameter by the second application module 350. In this embodiment, the second application module 350 provides a data analysis about the usage of medical apparatuses, which comprises for each considered medical apparatus a percentage of time in which the respective medical apparatus executes medical examinations. For example, if there are 20 medical examinations with a medical apparatus within a certain day, wherein only 18 examinations can be processed properly by the first application module 320 (which in this embodiment anonymizes the medical data records and stores the anonymized medical data records in the cloud), then the corresponding medical data analysis is: "77% usage (based on 18 analyzed of 20 total data records)". Here the addressee of the medical data analysis is informed that the usage statistics is not calculated based on the total number of examinations on this day, but only on the fraction of valid examinations.

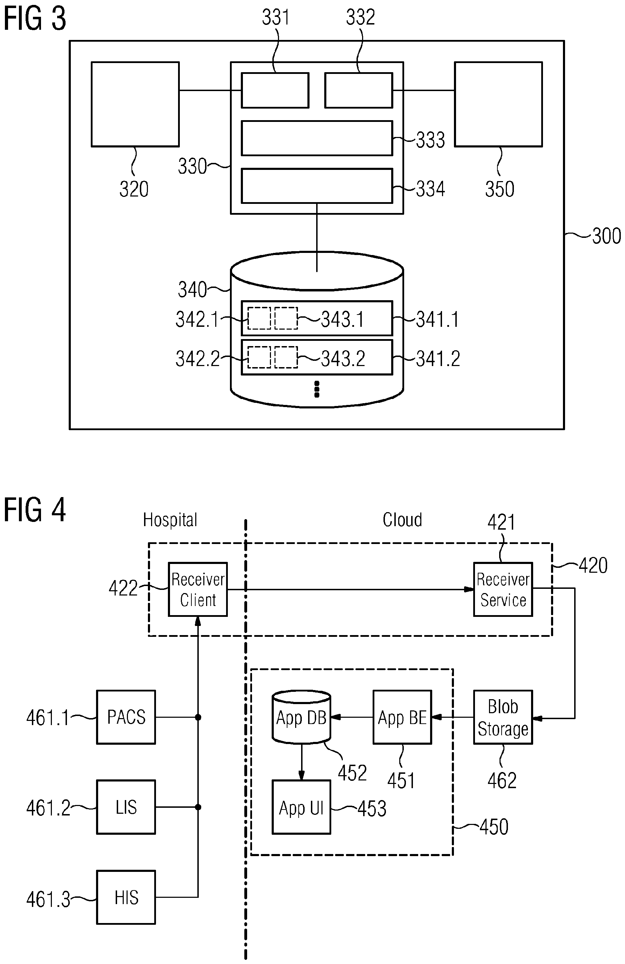

Fig. 3 shows a providing system 300 comprising a first application module 320, a failure analysis module 330 a failure database 340 and a second application module 350.

In this embodiment the first application module 320, the second application module 350 and the failure analysis module 330 are software applications within a cloud environment. Alternatively, the providing system 300, the first application module 320, the second application module 350 and/or the failure analysis module 330 can be hardware entities, e.g. (personal) computers, workstations, virtual machines running on a host hardware, microcontrollers, or integrated circuits. As an alternative, the providing system 300 and/or the failure analysis module 330 can be a real or a virtual group of computers (the technical term for a real group of computers is "cluster", the technical term for a virtual group of computers is "cloud").

The communication between the first application module 320, the second application module 350 and the failure analysis module 330 is in this embodiment executed via API ("Application Programming Interface") calls, alternatively the communication can be done via web-services or by providing data records in public available storages (accessable e.g. by "File Transfer Protocoll" FTP or "Hypertext Transfer Protocoll" HTTP).

In this embodiment, the failure analysis module 330 comprises an input interface 331, an output interface 332, a calculation module 333 and a storage module 334. In this embodiment, the input interface 331, the output interface 332, the calculation module 333 and the storage module 334 are embodied as software sub-modules of the failure analysis module 330, they can also be embodied as hardware sub-modules if the failure analysis module 330 is a hardware module.

An input interface 331 and an output interface 332 can be embodies as a hardware interface or as a software interface (e.g. PCI-Bus, USB or Firewire). In general, a calculation module 333 can comprise hardware elements and software elements, for example a microprocessor or a field programmable gate array. A hardware memory module 334 can be embodied as non-permanent main memory (e.g. random access memory) or as permanent mass storage (e.g. hard disk, USB stick, SD card, solid state disk).

In the displayed embodiment, the failure database 340 is embodied separately from the failure analysis module 330, e.g. as a database server or as a database service. For databases several standards are known, e.g. relational databases servers or services using e.g. SQL ("Structured Query Language"), or NoSQL database servers or services storing data e.g. in key-value-pairs or using graph models, only partially guaranteeing the consistency and the availability of the stored data records. Alternatively the failure database 340 can be embodied within the storage module 334 of the failure analysis module 330.

In this embodiment, the failure database 340 contains failure records 341.1, 341.2, each failure record 341.1, 341.2 comprising a module identifier 342.1, 342.2 and an apparatus identifier 343.1, 343.2 as metadata of the respective medical data record. If the failure database 340 is a relational database, it is favorable to use primary keys as module identifiers 342.1, 342.2 and apparatus identifiers 343.1, 343.2, and to store details of the modules and apparatuses in separate tables (to ensure a proper normalization of the database) with respect to the respective primary key.

Fig. 4 shows a graphical sketch of the functionality of a cloud framework for storing and processing medical data records comprising a hospital environment and a cloud environment without a failure analysis module. On the left side of Fig. 4 software and/or hardware modules are displayed which are parts of a local hospital environment; on the right hand side of Fig. 4 software and/or hardware modules are displayed with are parts of the cloud environment. Due to legal data privacy restrictions personal data may only be stored in the hospital environment, and not in a cloud environment.

The hospital environment comprises data sources 461.1, 461.2, 461.3, comprising a PACS 461.1 ("Picture Archiving and Communication System"), a LIS 461.2 ("Laboratory Information System") and a HIS 461.3 ("Hospital information System"). Furthermore, the hospital environment comprises a receiver client 422, which gathers data records from the data sources 461.1, 461.2, 461.3, anonymizes them and sends them to the cloud environment. The receiver client 422 can be local software installed on one or more of the data sources 461.1, 461.2, 461.3, in this embodiment it is embodied as a separate server located in the internal network of a hospital, so that it can access the the data sources 461.1, 461.2, 461.3.

The cloud environment comprises a receiver service 421 which receives the anonymized data records from the receiver client 422. In this embodiment, the receiver service 421, the receiver client 422 and their communication constitute the first application module 420. Furthermore, the cloud environment comprises a BLOB (acronym for "Binary Large Object") storage 462 which is embodied to store the medical data records received by the receiver service 421.

Furthermore, the cloud environment comprises a second application module 450, comprising an application backend 451, an application database 452 and an application frontend 453. The application backend 451 is embodied to access the BLOB storage 462 in order to provide a statistical analysis of the medical data records contained in the BLOB storage 462. The results of the statistical analysis are then stored in the application database 452, which can be accessed by the application frontend 453 in order to display the results of the statistical analysis.

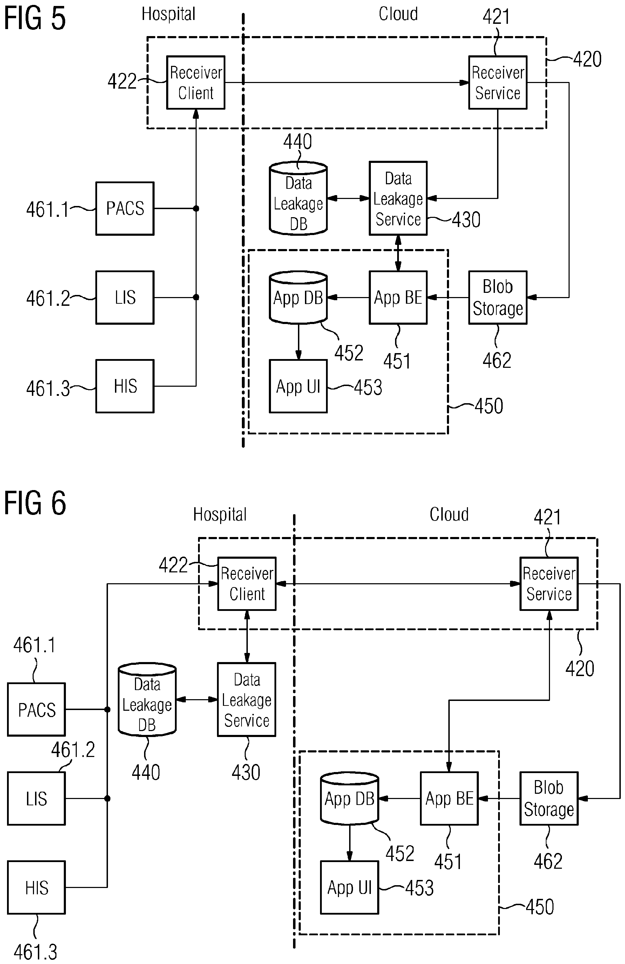

Fig. 5 shows a graphical sketch of the functionality of a cloud framework for storing and processing medical data records comprising a hospital environment and a cloud environment, wherein the cloud environment comprises a data leakage service 430 as a failure analysis module and a data leakage database 440 as a failure database. This cloud framework is an extension of the cloud framework depicted in Fig. 4.

In this embodiment, the data leakage service 430 is in communication with the receiver service 421 and with the application backend 451. The first application module 420, comprising the receiver service 421 and the receiver client 422, processes data records from the hospital data sources 461.1, 461.2, 461.3. Therein the receiver client 422 anonymizes the data records and sends the anonymized data records to the receiver service 421. The receiver service 421 checks the anonymized data records for compliance with predefined requirements, in this case that the duration of the medical examination lies in predefined boundaries. If the duration of the medical examination is within the predefined boundaries (and all other predefined checks are positive), the medical data record will be sent to the BLOB storage 462. If the duration of the medical examination is not within the predefined boundaries, the data record is considered as invalid, and a failure record 341.1, 341.2 comprising the data record is sent to the data leakage service 430 and stored in the data leakage database 440 by the data leakage service 430.

In this embodiment, the application backend 451 is embodied to provide a statistical analysis about the usage of one or several medical apparatuses, e.g. the relation of the total examination time to the operation time of the medical apparatus. Therefor the application backend 451 can communicate with the BLOB storage 462 and with the data leakage service 430. From the medical data records stored in the BLOB storage 462 the relation of the total examination time to the total operation time can be calculated, and by querying the data leakage service 430 with the medical apparatus, the number of failure records 341.1, 341.2 comprising the identifier of the respective medical apparatus can be obtained by the application backend 451. So the application frontend 453 can display the ratio of total examination time to the total operation time as well as the number of failure records 341.1, 341.2, where the number of failure records 341.1, 341.2 can be used for estimating the influence of erroneous medical data records on the statistical analysis.

In this embodiment, also the receiver client 422 can send failure records 341.1, 341.2 to the data leakage service 430 through the receiver service 421, e.g. if the anonymization of the medical data record is not successful. In this case it is advantageous to include only metadata of the medical data record to the data leakage service 430, wherein the metadata does not contain personal data of a patient, so that the failure records 341.1, 341.2 can be stored in the cloud environment without violating data privacy regulations.

Fig. 6 shows a graphical sketch of the functionality of a cloud framework for storing and processing medical data records comprising a hospital environment and a cloud environment, wherein the hospital environment comprises a data leakage service 430 as a failure analysis module and a data leakage database 440 as a failure database. This cloud framework is an extension of the cloud framework depicted in Fig. 4.

In this embodiment, the data leakage service 430 is only in communication with the receiver client 422. The application backend 451 can access the data leakage service 430 through the receiver service 421 and the receiver client 422. If the receiver client 422 cannot process a medical data record from one of the data sources 461.1, 461.2, 461.3 properly, because e.g. the anonymization procedure fails, it sends a failure record 341.1, 341.2 to the data leakage service 430, which stores the failure record 341.1, 341.2 in the data leakage database 440. By keeping the data leakage service 430 and the data leakage database 440 within the hospital environment, it is possible to store non-anonymized medical data records in the data leakage database 440 without violating data privacy constraints.

The data leakage service 430 can provide the failure parameter to the application backend 451 through the receiver client 422 and the receiver service 421. In this embodiment, the data leakage service 430 ensures that the failure parameter does not contain any personal data, alternatively the receiver client 422 can anonymize the failure parameter. If the failure parameter comprises e.g. the total number of failure records 341.1, 341.2, the application backend 451 or the application frontend 453 can use this total number to allow for estimating a user the impact of the erroneous data records on the statistical analysis.

By operating the data leakage service 430 and the data leakage database 440 within the hospital environment, it is possible that the failure record 341.1, 341.2 comprises the complete medical data record including personal data, without storing the personal data in the cloud. Using the complete medical data records is useful for locating the cause for the non-processability of the medical data record. For the error search, on the one hand, the failure records 341.1, 341.2 can be accessed directly from within the hospital environment; on the other hand, the failure records 341.1, 341.2 can also be accessed from within the cloud environment through the receiver service 421 and the receiver client 422, if certain authentication conditions are met (e.g. the user is authenticated as a technical administrator) and checked by the local receiver client 422 within the hospital environment.

In particular, the arrows in Fig. 4, Fig. 5 and Fig. 6 indicate the flow of data between the single components within the cloud framework.

## Claims

1. A method for providing a failure parameter, comprising the following steps:
- receiving (REC-1) a medical data record by a first application module (320, 420),
- determining (DET-1) a failure record (341.1, 341.2) by the first application module (320, 420), if the medical data record cannot be processed by the first application module (320, 420),
wherein the failure record (341.1, 341.2) is based on the data record,
- transmitting (TRM) the failure record (341.1, 341.2) from the first application module (320, 420) to a failure analysis module (330, 430),
- saving (SAV) the failure record (341.1, 341.2) in a failure database (340, 440) by the failure analysis module (330, 430),
- determining (DET-2) a failure parameter by the failure analysis module (330, 430),
wherein the failure parameter is based on a statistical analysis of the failure database (340, 440),
- providing (PROV-1) the failure parameter by the failure analysis module (330, 430).

2. A method for providing a data analysis, comprising all steps of claim 1, furthermore comprising the following steps:
- receiving (REC-2) the failure parameter by a second application module (350, 450),
- providing (PROV-2) a medical data analysis based on the failure parameter by the second application module (350, 450).

3. The method according to one of the preceding claims,
wherein the failure record (341.1, 341.2) comprises a failure type, wherein the failure type indicates the reason for the medical data record being non-processible by the first application module (320, 420).

4. The method according to one of the preceding claims,
wherein the medical data record comprises the result of a medical examination of a patient by a medical apparatus,
wherein the failure record (341.1, 341.2) is an anonymized data record.

5. The method according to one of the preceding claims,
wherein the medical data record comprises the result of a medical examination of a patient by a medical apparatus,
wherein the failure record (341.1, 341.2) comprises metadata,
wherein the metadata relates to the medical apparatus, a parameter of the medical examination and/or to the first application module (320, 420).

6. The method according to claim 5, wherein the metadata contains at least one of the following values of the medical examination:
- start time and/or end time of the medical examination,
- duration of the medical examination,
- input parameter of the medical examination,
- hardware components of the medical apparatus,
- software version of the medical apparatus,
- identifier of the first application module.

7. The method according to one of the preceding claims,
wherein the medical data record cannot be processed by the first application module (320, 420) due to the medical data record exhibiting a non-readable format or due to the medical data record being classified as inconsistent by the first application module (320, 420).

8. The method according to one of the preceding claims,
wherein the failure parameter relates to the number of failure records (341.1, 341.2) in the failure database (340, 440).

9. The method according to one of the preceding claims,
wherein the failure record (341.1, 341.2) comprises a module identifier (342.1, 342.2) of the first application module (320, 420),
and wherein the failure parameter comprises the number of failure records (341.1, 341.2) in the failure database (340, 440) comprising a given module identifier (342.1, 342.2).

10. The method according to one of the preceding claims,
wherein the medical data record comprises the results of an imaging medical examination by an medical apparatus,
wherein the failure record (341.1, 341.2) comprises an apparatus identifier (343.1, 343.2) of the medical apparatus,
and wherein the failure parameter comprises the number of failure records (341.1, 341.2) in the failure database (340, 440) comprising a given apparatus identifier (343.1, 343.2).

11. A providing system (300), comprising the following units:
- first application module (320, 420), embodied for receiving (REC-1) a medical data record,
furthermore embodied for determining (DET-1) a failure record (341.1, 341.2), if the medical data record cannot be processed by the first application module (320, 420), wherein the failure record (341.1, 341.2) is based on the data record,
furthermore embodied for transmitting (TRM) the failure record (341.1, 341.2) to a failure analysis module (330, 430)
- failure analysis module (330, 430), embodied for saving (SAV) the failure record (341.1, 341.2) in a failure database (340, 440),
furthermore embodied for determining (DET-2) a failure parameter, wherein the failure parameter is based on a statistical analysis of the failure database (340, 440),
furthermore embodied for providing (PROV-1) the failure parameter.

12. The providing system (300) according to claim 11, furthermore comprising the following unit:
- second application module (350, 450), embodied for receiving (REC-2) the failure parameter,
furthermore embodied for providing (PROV-2) a medical data analysis based on the failure parameter.

13. The providing system (300) according to claim 11 or claim 12, furthermore embodied to execute a method according to one of the claims 3 to 10.

14. A computer program product comprising a computer program, the computer program being loadable into a storage unit of a providing system (300), including program code sections to make the providing system (300) execute the method of any one of claims 1 to 10 when the computer program is executed in said providing system (300).

15. A computer-readable medium, on which program code sections of a computer program are saved, said program code sections being loadable into and/or executable in a providing system (300) to make the providing system (300) execute the method of any one of the claims 1 to 10 when the program code sections are executed in said providing system (300).

## Amended claims

### Amended claims in accordance with Rule 137(2) EPC.

1. A method for providing a data analysis, comprising the following steps:
- receiving (REC-1) a medical data record by a first application module (320, 420),
wherein the medical data record comprises the result of a medical examination of a patient by a medical apparatus,
- determining (DET-1) a failure record (341.1, 341.2) by the first application module (320, 420), if the medical data record cannot be processed by the first application module (320, 420),
wherein the failure record (341.1, 341.2) is based on the data record, and wherein the failure record (341.1, 341.2) is an anonymized data record,
- transmitting (TRM) the failure record (341.1, 341.2) from the first application module (320, 420) to a failure analysis module (330, 430),
- saving (SAV) the failure record (341.1, 341.2) in a failure database (340, 440) by the failure analysis module (330, 430),
- determining (DET-2) a failure parameter by the failure analysis module (330, 430),
wherein the failure parameter is based on a statistical analysis of the failure database (340, 440),
- providing (PROV-1) the failure parameter by the failure analysis module (330, 430).
- receiving (REC-2) the failure parameter by a second application module (350, 450),
- providing (PROV-2) a medical data analysis based on the failure parameter by the second application module (350, 450) .

2. The method according to one of the preceding claims,
wherein the failure record (341.1, 341.2) comprises a failure type, wherein the failure type indicates the reason for the medical data record being non-processible by the first application module (320, 420).

3. The method according to one of the preceding claims,
wherein the medical data record comprises the result of a medical examination of a patient by a medical apparatus,
wherein the failure record (341.1, 341.2) comprises metadata,
wherein the metadata relates to the medical apparatus, a parameter of the medical examination and/or to the first application module (320, 420).

4. The method according to claim 3, wherein the metadata contains at least one of the following values of the medical examination:
- start time and/or end time of the medical examination,
- duration of the medical examination,
- input parameter of the medical examination,
- hardware components of the medical apparatus,
- software version of the medical apparatus,
- identifier of the first application module.

5. The method according to one of the preceding claims,
wherein the medical data record cannot be processed by the first application module (320, 420) due to the medical data record exhibiting a non-readable format or due to the medical data record being classified as inconsistent by the first application module (320, 420).

6. The method according to one of the preceding claims,
wherein the failure parameter relates to the number of failure records (341.1, 341.2) in the failure database (340, 440) .

7. The method according to one of the preceding claims,
wherein the failure record (341.1, 341.2) comprises a module identifier (342.1, 342.2) of the first application module (320, 420),
and wherein the failure parameter comprises the number of failure records (341.1, 341.2) in the failure database (340, 440) comprising a given module identifier (342.1, 342.2).

8. The method according to one of the preceding claims,
wherein the medical data record comprises the results of an imaging medical examination by an medical apparatus,
wherein the failure record (341.1, 341.2) comprises an apparatus identifier (343.1, 343.2) of the medical apparatus,
and wherein the failure parameter comprises the number of failure records (341.1, 341.2) in the failure database (340, 440) comprising a given apparatus identifier (343.1, 343.2).

9. A providing system (300), comprising the following units:
- first application module (320, 420), embodied for receiving (REC-1) a medical data record, wherein the medical data record comprises the result of a medical examination of a patient by a medical apparatus,
furthermore embodied for determining (DET-1) a failure record (341.1, 341.2), if the medical data record cannot be processed by the first application module (320, 420), wherein the failure record (341.1, 341.2) is based on the data record, and wherein the failure record (341.1, 341.2) is an anonymized data record,
furthermore embodied for transmitting (TRM) the failure record (341.1, 341.2) to a failure analysis module (330, 430)
- failure analysis module (330, 430), embodied for saving (SAV) the failure record (341.1, 341.2) in a failure database (340, 440),
furthermore embodied for determining (DET-2) a failure parameter, wherein the failure parameter is based on a statistical analysis of the failure database (340, 440),
furthermore embodied for providing (PROV-1) the failure parameter,
- second application module (350, 450), embodied for receiving (REC-2) the failure parameter,
furthermore embodied for providing (PROV-2) a medical data analysis based on the failure parameter.

10. The providing system (300) according to claim 9, furthermore embodied to execute a method according to one of the claims 2 to 8.

11. A computer program product comprising a computer program, the computer program being loadable into a storage unit of a providing system (300), including program code sections to make the providing system (300) execute the method of any one of claims 1 to 8 when the computer program is executed in said providing system (300).

12. A computer-readable medium, on which program code sections of a computer program are saved, said program code sections being loadable into and/or executable in a providing system (300) to make the providing system (300) execute the method of any one of the claims 1 to 8 when the program code sections are executed in said providing system (300).
